(19) **Europäisches Patentamt**
**European Patent Office**
**Office européen des brevets**

(11) **EP 1 389 140 B1**

(12) **EUROPÄISCHE PATENTSCHRIFT**

(45) Veröffentlichungstag und Bekanntmachung des Hinweises auf die Patenterteilung:
**17.02.2010 Patentblatt 2010/07**

(21) Anmeldenummer: **02771647.1**

(22) Anmeldetag: **16.05.2002**

(51) Int Cl.:
*A61M 1/30* *(2006.01)*  *A61M 1/34* *(2006.01)*
*A61M 5/142* *(2006.01)*  *A61M 1/16* *(2006.01)*
*F04B 23/04* *(2006.01)*

(86) Internationale Anmeldenummer:
**PCT/EP2002/005407**

(87) Internationale Veröffentlichungsnummer:
**WO 2002/094349 (28.11.2002 Gazette 2002/48)**

(54) **VERFAHREN UND VORRICHTUNG ZUM BILANZIEREN VON FLÜSSIGKEITEN**

METHOD AND DEVICE FOR BALANCING LIQUIDS

PROCEDE ET DISPOSITIF DESTINES A EQUILIBRER DES LIQUIDES

(84) Benannte Vertragsstaaten:
**AT BE CH CY DE DK ES FI FR GB GR IE IT LI LU MC NL PT SE TR**

(30) Priorität: **21.05.2001 DE 10124951**

(43) Veröffentlichungstag der Anmeldung:
**18.02.2004 Patentblatt 2004/08**

(73) Patentinhaber: **Miltenyi Biotec GmbH**
**51429 Bergisch Gladbach (DE)**

(72) Erfinder:
• **SCHIMMELPFENNIG, Winfried**
**18292 kKrakow am See (DE)**

• **LANTOW, Holger**
**18146 Rostock (DE)**

(74) Vertreter: **Grünecker, Kinkeldey, Stockmair & Schwanhäusser Anwaltssozietät Leopoldstrasse 4 80802 München (DE)**

(56) Entgegenhaltungen:
**EP-A- 0 834 329     DE-A- 3 328 744**
**DE-A- 3 439 661     DE-A- 19 856 744**
**FR-A- 2 368 963**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zum Bilanzieren von Flüssigkeiten und eine darauf aufbauende Vorrichtung für eine medizinische Behandlung.

Bei vielen extrakorporalen Blutbehandlungs- oder Austauschverfahren (z.B. Hämofiltration, Plasmaaustausch, ...) wird einem Patienten während der Behandlung kontinuierlich Flüssigkeit aus seinem Blut abfiltriert und durch eine Substituatlösung ersetzt. Hierbei tritt das Problem auf, dem Patienten genau so viel Substituat zuzuführen, wie ihm gleichzeitig durch das Filter hindurch entzogen wird. Bei der Hämodialyse wird Blut in einem Dialysator an einer Membran vorbeigeleitet, die eine Diffusion von niedermolekularen Giftstoffen in eine auf der anderen Membranseite entlangfließende wässrige Salzlösung (Dialysat) ermöglicht.

Da bei diesen Filterverfahren die Membranen für Wasser durchlässig sind, besteht die Gefahr, dass der Patient durch osmotische oder andere transmembrane Druckdifferenzen während der Therapie allmählich be- oder entwässert wird. Daher ist in allen modernen Geräten zur Blutbehandlung eine Bilanzierung des Dialysat- bzw. Filtrat/Substituat-Kreislaufs vorhanden. Die ausreichende Genauigkeit dieser Volumenbilanz über die gesamte Therapiezeit ist eine Grundvoraussetzung für die Sicherheit des Patienten.

Wegen der z.T. großen Austauschmengen bzw. Dialysatmengen (mehr als 100 Liter Dialysat pro Behandlung sind üblich) besteht bei den genannten Verfahren die Notwendigkeit der exakten Bilanzierung von entzogener Flüssigkeit zur zugeführten (übliche tolerierte Abweichung bis max. 0,3 Liter) über die gesamte Behandlungszeit. Zum Stand der Technik gehören gravimetrische und volumetrische Bilanziersysteme.

Bei gravimetrischen Verfahren werden Waagen zur Erfassung von Gesamtvolumina (Filtrat + Substituat = const., Bilanzwaage, z.B. BRAUN Diapact CRRT) oder als getrennte Filtrat- und Substituatwaagen angewandt (Hospal Prisma, Gambro AK100, Sartorius Haemoprocessor). Diese Lösungen sind aufwendig, setzen eine zumindest portionsweise wägbare Bereitstellung von Substituatlösung voraus, sind störanfällig und zyklisch kalibrierpflichtig.

Bei volumetrischen Bilanzierprinzipien kann man zwischen fest im Gerät installierten Bilanzkammern, die zwischen verschiedenen Behandlungen aufwendig gereinigt und desinfiziert werden müssen (Fresenius US04267040, EP00867195A1), und solchen im single-use-Disposable unterscheiden. Letztere arbeiten direkt als Bestandteil des Patienten-Schlauchsystems in nur einer Behandlung und werden danach zusammen mit dem gesamten Set entsorgt. Die Vorbereitungszeit der Bilanzierungsvorrichtung zum nächsten Patienten und die Infektions- und Keimverschleppungsgefahr entfallen hier.

Übliche bilanzierende Disposables arbeiten mit kalibrierten volumenstarren Kammern im Schlauchset, die zylisch über Umschaltventile gefüllt und geleert werden und deren Volumenkonstanz und Genauigkeit im Umschaltmoment den Bilanzfehler bestimmt (z.B. LKB, Pat. US 3620215). Eine neuere Lösung beschreibt eine dreilagige Folienbilanzkammer, die über ein von außen starr konstantgehaltenes Kammergesamtvolumen eine zyklische Bilanzierung der beiden flexibel aneinanderliegenden Innenkammern ermöglicht (Fresenius, Folienkammer US 5836908).

Diese Kammer ermöglicht nur eine zyklische Bilanzierung (Füllen/Leeren/Füllen/Leeren), die Serienfertigung ist durch ihre Dreilagigkeit aufwendig und ihr Füllvolumen ist relativ zum gesamten extrakorporalen Füllvolumen des Schlauchsets hoch, um die notwendige Anzahl der Umschaltzyklen gering zu halten, da sich der Gesamtbilanzfehler aus der Summe der Einzelfehler jedes einzelnen Umschaltzyklus (Ventilspiel, eingeschlossene Luftblasen...) ergibt.

Ein weiteres Bilanzierprinzip wird in Pat. FR 2368963 (Fig. 2) und identisch in Pat. DE 3439661 (Fig. 2) beschrieben, welches mit zwei Pumpen arbeitet, die am Eingangs- und Ausgangsanschluss eines Dialysators synchron fördern und die zyklisch von Ventilgruppen mit je vier Ventilen gegeneinander vertauscht werden. Dadurch sollen sich ihre Förderfehler in der Summe über den Therapiezeitraum aufheben. Grundvoraussetzung dieses Prinzips ist jedoch die absolute Förderkonstanz der eingesetzten Pumpen, die in DE 3439661 mit besser 1 % gefordert wird, also die Unabhängigkeit von den verschiedenen und wechselnden Umgebungsbedingungen der beiden Pumppositionen im Dialysatfluss. Daher wird im Patenttext eine zumindest werksseitige Kalibrierung der Präzisionspumpen empfohlen. Schlauchpumpen schließen sich wegen ihrer geringeren Förderkonstanz damit ohnehin aus, ein Disposable hat nach diesem Prinzip nicht die geforderte kalibrierte Genauigkeit. Der ebenfalls empfohlene Einsatz von zwei induktiven Durchflusssensoren zur Verbesserung der Förderkonstanz ist in einem Disposable ebenfalls als Wegwerfartikel zu teuer und ansonsten wegen ihrer bei jeder Therapie immer wieder notwendigen Desinfektion und Sterilität undiskutabel aufwendig.

Die EP 0 834 329 A1 offenbart ein Verfahren und eine Vorrichtung zur Behandlung von Blut, wobei die Flussrate eine Pumpe verringert wird, wenn während der Blutbehandlung Druck aufgebaut werden sollte, um den Druckaufbau zu beseitigen und normalen Zustand so schnell wie möglich wieder herzustellen. Nachdem der Druckaufbau beseitigt wurde, wird die Flussrate der Pumpe wieder auf die ursprüngliche Rate beschleunigt, ohne aber einen erhöhten Druck durch eine abrupte Beschleunigung der Pumpenflussrate aufzubauen.

Der Erfindung liegt die Aufgabe zugrunde, ein Bilanzierdisposable zur kontinuierlich arbeitenden Bilanzierung zweier Flüssigkeiten für eine medizinische Behandlungsvorrichtung zu schaffen, welches ohne volumenstarre Kammern auskommt, sich einfach handhaben und kostengünstig als Einmalartikel herstellen lässt und bei der die üblicherweise geforderte Bilanziergenauigkeit ohne Kalibrierungen erreicht wird.

Diese Aufgabe wird mit Hilfe eines Verfahrens mit den Merkmalen des Anspruches 1 und einer Vorrichtung mit den Merkmalen des Anspruches 4 gelöst. Vorteilhafte Ausgestaltungen sind Gegenstand der jeweiligen Unteransprüche.

Werden die beiden zu bilanzierenden Flüssigkeitsströme parallel durch zwei separate synchrom laufende Pumpen angetrieben, so entsteht eine wachsende Volumenfehlbilanz, die sich aus der unvermeidlichen Differenz der Förderraten und der verstrichenen Zeit ergibt. Werden nun die beiden Pumpen gegeneinander vertauscht, so entsteht bei konstant gehaltenen Pumpbedingungen der gleiche Bilanzfehler mit umgekehrtem Vorzeichen. In der Summe beider Zyklen kompensiert er sich also genau zu Null,

$$(A\text{-}B) + (B\text{-}A) = 0,$$

diese Gleichung gilt selbst für sehr verschiedene konstantgehaltene Flussraten A und B, die Pumpen müssen also nicht kalibriert sein.

Wenn die Flussdifferenz in beiden Zyklen jedoch nicht gleich ist, weil irgendein Einflussparameter zeitlich driftet, sei es eine Alterung einer Pumpe oder eine Temperaturdrift eines regelnden Sensors einer Pumpe, so entsteht die Driftdifferenz als Fehlbilanz in der Gesamtsumme. Dies kann man jedoch ebenfalls kompensieren, indem man an den oben beschriebenen Doppelzyklus einen zweiten anschließt, der zeitlich genau entgegengesetzt abläuft. Bei konstanter Drift entsteht so die Fehlbilanz nun mit entgegengesetztem Vorzeichen und kompensiert so die Drift des ersten Doppelzyklus.

Also Pumpe 1 läuft im ersten Zyklus erst im Kanal A, dann im Kanal B, im nächsten Zyklus erst im Kanal B, dann im Kanal A..

Es lässt sich nachweisen, dass hier eine Systematik wirkt:

 1. Wenn man zwei Pumpen zyklisch austauscht, also spiegelt, so heben sich konstante Flussdifferenzen auf, (A-B) + (B-A) = 0. Flussratendriften wirken hierbei jedoch noch als Bilanzfehler.

 2. Wenn man diesen Zyklus danach in entgegengesetzter zeitlicher Reihenfolge ausführt, also spiegelt (A-B)+(B-A) + (B-A)+(A-B), so heben sich in der Summe auch die linearen Anteile der Driften gegeneinander auf.

 3. Durch weitere Spiegelungen (A-B)+(B-A)+(B-A)+ (A-B) + (B-A)+(A-B)+(A-B)+(B-A) ... wird praktisch jeder zeitlich höherpotente Driftanteil der Pumpenflussraten allmählich zu Null kompensiert.

Der Genauigkeitsunterschied lässt sich anhand einer zeitlich linear steigenden oder fallenden Drift x (t) der Pumpflussraten gut veranschaulichen (Flussrate von A ist beispielsweise linear um 1 x pro Zyklus ansteigend). Hätte man beispielsweise in diesem Fall einfach immer nur zyklisch die Kanäle gewechselt:

$$(A\text{-}B)+(B\text{-}A)+(A\text{-}B)+(B\text{-}A)$$

so wäre schon durch diese lineare Drift in der Summe ein Bilanzfehler von -2x entstanden:

$$1x - 2x + 3x - 4x = \text{-}2x.$$

Durch die Spiegelung

$$(A\text{-}B)+(B\text{-}A)+(B\text{-}A)+(A\text{-}B)$$

wird die Drift zeitlich stattdessen nun komplett kompensiert:

$$1x - 2x - 3x + 4x = 0!$$

Das erfindungsgemäße Bilanzierdiposable kann vorteilhaft als medizinisches Schlauchsystem entsprechend DE 198 56 744 ausgeführt werden und ermöglicht den Einsatz von in der Medizintechnik üblichen Rollenpumpen für die Volumenförderung in beiden bilanzierten Förderkanälen. Die Zuführung und die Umschaltung der Medien erfolgt vorzugsweise ebenfalls über Rollenpumpen, die eine definierte Regelung der Drücke auf den zu- und abfördernden Anschlüssen der beiden erstgenannten flussbestimmenden Pumpen ermöglicht. Diese Regelung ist notwendig, wenn die Eingangs- und Ausgangsdrücke der Bilanziereinheit nicht ohnehin prozessbedingt über eine komplette Bilanzierphase konstant und gleich sind.

Vorteilhaft kommt hier eine Schlauchfolienkassette nach Pat. DE19856744 zum Einsatz, welche die Kombination von nebeneinander angeordneten Rollenpumpen in einer geprägten Schlauchkassette beinhaltet.

Bei dem erfindungsgemäßen Bilanzierdisposable wird die volumetrische Bilanz durch wechselweise Umschaltung zweier Pumpenstrecken in einem zeitlich symmetrischen Raster erreicht. Dadurch werden in der Gesamtbilanz über einen entsprechend größeren Zeitraum Förderabweichungen in beiden Pumpenstrecken kompensiert.

Mittels geeigneter Umschaltstrategien (Spiegelverfahren) ist es so möglich, eine zeitliche Veränderung der Förderleistungen der beiden Pumpenstrecken komplett zu kompensieren.

Die Sicherung konstanter Eingangs- und Ausgangsdruckbedingungen der im Kanal flussbestimmenden zentralen Bilanzierpumpen kann wie folgt gesichert werden. Einerseits regelt die am jeweiligen Eingang aktive Eingangspumpe den Druck zur gerade flussbestimmenden Pumpe dieses Kanals auf einen konstanten Wert, indem jeweils ein Drucksensor, der zwischen den beiden Pumpen angeordnet ist, die Flussrate der gerade aktiven Eingangspumpe so steuert, dass die Eingangsdruckbedingungen der im Kanal flussbestimmenden Pumpe in beiden Zyklen im Mittel konstant bleiben. Andererseits werden auch die im Zyklus gerade aktiven ausgangsseitigen Pumpen durch vorgeschaltete Drucksensoren an ihren Eingängen so in der Flussrate gesteuert, dass auch die Ausgangsdruckbedingungen der flussbestimmenden zentralen Pumpen in beiden Zyklen im Mittel konstant gehalten werden.

Schließlich kann durch eine gezielte Ansteuerung der Eingangspumpe des einen Kanals und der Ausgangspumpe des anderen Kanals durch eine zentrale Pumpe hindurch eine Überkreuzförderung erreicht werden, die in allen Stoffaustauschanwendungen einer direkten Bypassfunktion entspricht, da sie dem Prozess ebenfalls genau die Menge Flüssigkeit zurückgibt, die auch entnommen wurde, jedoch ohne sie dabei auszutauschen.

Das erfindungsgemäße Disposable ermöglicht durch seine sehr kompakte Ausführung ohne zusätzliche volumetrische Kammern eine sehr geringe extrakorporale Flüssigkeitsfiillmenge. Als vorteilhaft erweist sich auch die sehr flache Bauweise, die es erlaubt, das flexible Schlauchsystem als sterile Einheit in einer flachen Kunststoffumhüllung zu verpacken, die gegen Stoß unempfindlich ist und sich leicht stapeln und transportieren lässt, da sie keine starren Kammern enthält.

Vorzugsweise besteht die Komponente des Bilanzierdisposables aus zwei Folien, die unter Ausprägung der Pumpkanäle und der notwendigen Verbindungsleitungen miteinander verschweißt sind. Diese Folien beinhalten vorzugsweise auch alle weiteren, für eine Behandlung notwendigen Pumpkanäle, sowie die Druckkammern für die Drucksensorik (DE19856744). Es läßt sich auf einfache Weise in einem einzigen Arbeitsvorgang kostengünstig herstellen.

Da das Disposable nun auch sämtliche Komponenten der Bilanzierungsvorrichtung umfasst und einfacher Bestandteil eines umfassenderen Schlauchsystems z.B. zur extrakorporalen Blutbehandlung sein kann, ergibt sich so eine sinnvolle Erweiterung bestehender Systeme, wenn in einer Therapie eine Bilanzierung von Flüssigkeiten erforderlich wird.

Nachfolgend wird unter Bezugnahme auf die Zeichnungen eine Ausführungsform des Bilanzierdisposables erläutert. Dabei zeigen:

Fig. 1     das schematische Prinzip der Bilanzierung anhand von insgesamt 10 Pumpen

Fig. 2 und 3     ein medizinisches Behandlungsverfahren, den Therapeutischen Plasmaaustausch, unter Anwendung des erfindungsgemäßen Bilanzierungsverfahrens.

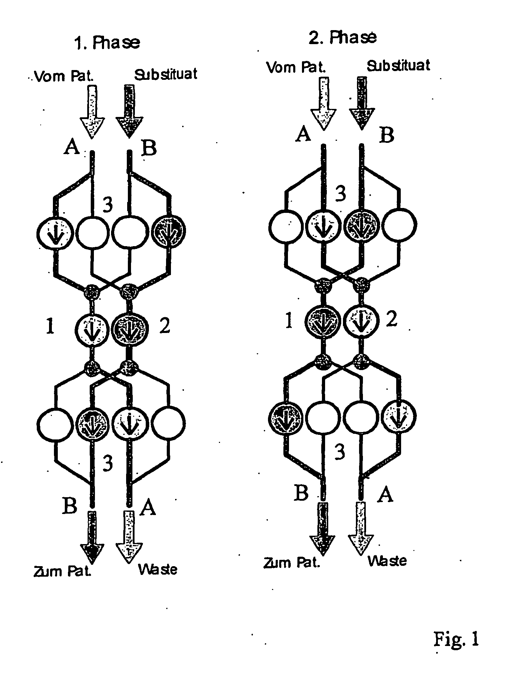

In Fig. 1 wird das Flussschema der Bilanzierung in beiden Phasen dargestellt, die zyklisch in zeitlicher Abfolge wechseln. Die beiden zu bilanzierenden Flüssigkeitsströme bzw. Pumpkanäle A und B werden durch zwei separate, synchron laufende zentrale Pumpen 1, 2 (Bilanzierpumpen) angetrieben. Die Eingangs- und Ausgangspumpen sind mit 3 bezeichnet, sie umfassen vier Eingangs- und vier Ausgangspumpen. Hierbei laufen die beiden zentralen Pumpen 1, 2 mit konstanter Förderrate durch, während jeweils eine von zwei Eingangs- und eine von zwei Ausgangspumpen ihre Zuordnung zu einem Pumpkanal A, B definiert und dabei über Drucksensoren in der Förderrate geregelt die Druckverhältnisse der zentralen Pumpen 1, 2 konstant halten. So ist in beiden Phasen gewährleistet, dass der Flüssigkeitsstrom der Gesamtanordnung in beiden Phasen den gleichen Eingang mit dem dazugehörigen Ausgang verbindet, jedoch die zentralen Pumpen 1, 2 ihre Kanalzuordnung tauschen, ohne dass sich ihre individuellen Pumpbedingungen ändern. Dadurch werden Abweichungen der beiden Pumpenflussraten gegeneinander kompensiert und ein kontinuierlicher Gesamtfluss mit einer ausgeglichenen Volumenbilanz in der Therapie ermöglicht.

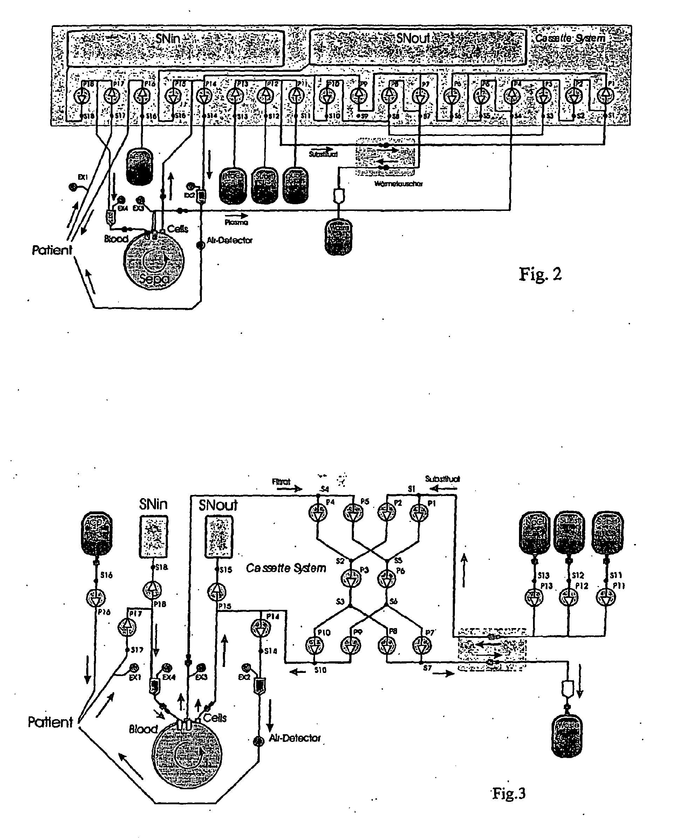

Fig. 2 und 3 zeigen ein medizinisches Behandlungsverfahren, den Therapeutischen Plasmaaustausch, in dem das beschriebene Bilanzierverfahren angewandt wird. Hierbei zeigt Fig.3 das Flussschema mit allen Pumpen, P1 .. P18, und Sensoren, S1 ..S18, dem Blutseparator, der aus dem Patientenblut kontinuierlich ein Filtrat gewinnt, das durch ein Substituat bilanziert ersetzt wird, und allen hierzu notwendigen Flüssigkeitsbeuteln. In Fig. 2 wird das konkrete Kassettenlayout der für dieses Schema entwickelten kreuzungsfreien Pumpenkassette dargestellt. Die Pumpennummem entsprechen in beiden Darstellungen der räumlichen

Anordnung in dieser Kassettenlösung. Hierbei bilden die Pumpen P1 .. P10 das Bilanziermodul. Durch die räumliche Nähe der direkt nebeneinander angeordneten Pumpen in der Kassette ist das Gesamtvolumen des Bilanziermoduls minimal. Die Anwendung bezieht sich speziell auf die Schlauchkassettenlösung nach DE19856744. Dort werden zwei Anwendungsbeispiele beschrieben, die Adsorbertechnologien zur Blutreinigung verwenden. Diese dort beschriebenen Adsorber sind durch ein definiertes Füllvolumen gekennzeichnet, sind also volumenstarre Gebilde, die keine Bilanzierung von Ein- und Ausgangsflüssen erfordern. Durch das nun vorgestellte Bilanzierverfahren wird die Anwendungsbreite des o.g. Patents wesentlich erweitert, es sind nun alle Austausch- und Dialyseverfahren ohne weitere gerätetechnische Bilanziervorrichtungen in bilanzierter Form anwendbar.

Alle Pumpen werden durch je eine Kassettenpumpe repräsentiert. Die erreichten Genauigkeiten der Bilanzierung liegen auch bei stark wechselnden Bedingungen im Mittel bei 0,1% des geförderten Gesamtvolumens der Therapie. Zur Regelung der Eingangs- und Ausgangsdrücke an den beiden eigentlichen flussbestimmenden Bilanzpumpen werden die in der Kassette vorgegebenen Drucksensoren S2, S3, S5 und S6 benutzt. Die Genauigkeit dieser Sensoren ist ausreichend, da sie nur dazu benutzt werden einen definierten Druckzustand konstant zu halten, Nichtlinearitäten und Driften werden vom beschriebenen Bilanzierverfahren prinzipiell kompensiert. Die vier Eingangspumpen der Bilanziervorrichtung nach Fig.1 sind in der Reihenfolge von links nach rechts im Flußplan nach Fig.2 durch die Kassettenpumpen P4, P5, P2, P1 repräsentiert, die mittleren flussbestimmenden Bilanzpumpen durch P3 und P6, die vier unteren Ausgangspumpen durch P10, P9, P8 und P7.

Die Pumpen P11, P12 und P13 ermöglichen die Auswahl oder Mischung aus drei verschiedenen Substituatlösungen beim Plasmaaustausch, P16 dient der Antikoagulanz des extrakorporalen Blutvolumens mittels ACD-A, die Pumpen P14 und P17 befördern das Blut des Patienten zum Plasmaseparator und wieder zurück, P15 und P18 ermöglichen den zyklischen sogenannten Single-Needle-Betrieb mit nur einer Kanüle unter Nutzung der beiden integrierten SN-Kammem der Kassette.

**Patentansprüche**

1. Verfahren zur bilanzierten Förderung zweier Flüssigkeitsmengen mittels einer Gesamtanordnung mit je zwei Ein-und Ausgängen und zwei Flüssigkeitskanälen (A, B), weiche je einen Eingang mit dem dazugehörigen Ausgang verbinden, wobei in jedem der beiden Flüssigkeitskanäle (A, B) eine flurssbestimmende Bilanzierpumpe (1, 2) angeordnet ist, **dadurch gekennzeichnet, dass** an jedem Eingang und jedem Ausgang dieser beiden Bilanzierpumpen (1, 2) Jeweils zwei druckgeregelte Pumpen (3) angeordnet und derart miteinander verbunden und wechselseitig gesteuert sind, dass die Bilanzierpumpen (1, 2) entweder den Fluss in dem einen oder in dem anderen Kanal bestimmen und zyklisch entweder von dem einen oder von dem anderen Eingang der Gesamtanordnung wahlweise zu dem einen oder zu dem anderen Ausgang der Gesamtanordnung fördern, wobei die druckgeregelten Pumpen (3) hierbei die Eingangs- und Ausgangsdrücke der Bilanzierpumpen (1, 2) konstant halten.

2. Verfahren nach Anspruch 1, **gekennzeichnet durch** die schematische zyklische Umschaltung der Kanalzuordnungen in einem Raster zeitgleicher oder volumengleicher Intervalle.

3. Verfahren nach Anspruch 1, **gekennzeichnet durch** die Spiegelung aller zuvor aufsummierter Bilanzfehler und damit deren zeitliche Kompensation, indem jede einzelne Kanalumschaltung in einem festen Raster so ausgeführt wird, dass die Kanalzuordnungen aller zeitlich davor liegenden Umschaltungen nun noch einmal in gleicher Reihenfolge, nur jeweils mit vertauschten Kanälen, ausgeführt werden.

4. Vorrichtung zur Durchführung des Verfahrens nach Anspruch 1 umfassend eine Gesamtanordnung mit je zwei Ein- und Ausgängen und zwei Flüssigkeitskanälen (A, B), welche je einen Eingang mit dem dazugehörigem Ausgang verbinden, wobei, in jedem der beiden Flüssigkeitskanäle (A, B) eine flussbestimmende Bilanzierpumpe (1, 2) angeordnet ist, **dadurch gekennzeichnet, dass** an jedem Ein- und an jedem Ausgang dieser Bilanzierpumpen (1, 2) jeweils zwei druckgeregelte Pumpen (3) angeordnet und derart miteinander verbunden und wechselseitig gesteuert sind, dass die flussbestimmenden Bilanzierpumpen (1, 2) im Betrieb umschaltbar den Fluss entweder in dem einen oder in dem anderen Kanal bestimmen und zyklisch entweder von dem einen oder von dem anderen Eingang der Gesamtanordnung wahlweise zu dem einen oder anderen Ausgang der Gesamtanordnung fördern, wobei die durckgeregelten Pumpen (3) hierbei die Eingangs- und Ausgangsdrücke der Bilanzierpumpen (1, 2) konstant halten.

5. Vorrichtung zur Durchführung des Verfahrens nach Anspruch 1, **dadurch gekennzeichnet, dass** die Pump-, Verbindungs- und Umschaltkanäle der Flüssigkeitskanäle (A, B) als flexibles Schlauchsystem ausgeführt und als Bilanzierpumpen (1, 2) Rollenpumpen vorgesehen sind.

6. Vorrichtung nach Anspruch 4, **dadurch gekennzeichnet dass** im Betrieb die am jeweiligen Eingang aktive Eingangspumpe den Druck zur gerade flussbestimmenden Bilanzierpumpe (1 bzw. 2) dieses Kanals auf einen konstanten Wert regelt, indem jeweils ein Drucksensor, der zwischen den beiden Pumpen angeordnet ist, die Flussrate der gerade aktiven Eingangspumpe steuert, um die Eingangsdruckbedingungen der im Kanal flussbestimmenden Bilanzierpumpe (1, 2) in beiden Zyklen gleich und konstant zu halten.

7. Vorrichtung nach Anspruch 4 und 6, **gekennzeichnet dadurch, dass** im Betrieb auch die im Zyklus gerade aktiven ausgangsseitigen Pumpen (3) durch vorgeschaltete Drucksensoren an ihren Eingängen gesteuert werden, so dass auch die Ausgangsdruckbedingungen der flussbestimmenden Bilanzierpumpen (1, 2) in beiden Zyklen gleich und konstant gehalten werden.

8. Vorrichtung nach Anspruch 4, **gekennzeichnet dadurch, dass** im Betrieb durch eine gezielte Ansteuerung der Eingangspumpe des einen Kanals und der Ausgangspumpe des anderen Kanals durch eine Bilanzierpumpe hindurch eine Überkreuzförderung erreicht wird, die in allen Stoffaustauschanwendungen einer direkten Bypassfunktion entspricht, da sie dem Prozess genau die Flüssigkeit zurückgibt, die auch entnommen wurde, ohne sie auszutauschen.

## Claims

1. Method for balanced pumping of two quantities of liquid by means of an overall arrangement which comprises two inputs and two outputs and two liquid channels (A, B), each of which connects an input to the associated output, wherein arranged in each of the two liquid channels (A, B) is a flow-determining balancing pump (1, 2), **characterised in that** arranged at each input and each output of these two balancing pumps (1, 2) are two pressure-regulated pumps (3) which are connected to one another and reciprocally controlled, such that the balancing pumps (1, 2) determine the flow in either one or the other channel and cyclically pump from one or the other input of the overall arrangement selectively to one or the other output of the overall arrangement, wherein the pressure-regulated pumps (3) keep the input and output pressures of the balancing pumps (1, 2) constant.

2. Method according to claim 1, **characterised by** the schematic cyclic switching over of the channel allocation in a grid-pattern of equal-time or equal-volume intervals.

3. Method according to claim 1, **characterised by** the mirroring of all the previously accumulated balancing errors and thus their temporal compensation in that each individual channel switch-over is carried out in a fixed grid-pattern such that the channel allocation of all the temporally previous switch-overs are now carried out again in the same sequence, but with the channels interchanged.

4. Device for carrying out the method according to claim 1, comprising an overall arrangement with two inputs and two outputs and two liquid channels (A, B) each of which connects an input to the associated output, wherein a flow-determining balancing pump (1, 2) is arranged in each of the two liquid channels (A, B), **characterised in that** arranged at each input and at each output of said balancing pumps (1, 2) are two pressure-regulated pumps (3), which are connected to one another and reciprocally controlled such that, during operation, the flow-determining balancing pumps (1, 2) determine the flow in switchable manner either in the one or the other channel and cyclically pump said flow from either one or the other input of the overall arrangement selectively to one or the other output of the overall arrangement, wherein the pressure-regulated pumps (3) keep the input and output pressures of the balancing pumps (1, 2) constant.

5. Device for carrying out the method according to claim 1, **characterised in that** the pump channels, connecting channels and switch-over channels of the liquid channels (A, B) are configured as a flexible hose system and that roller pumps are provided as the balancing pumps (1, 2).

6. Device according to claim 4, **characterised in that**, during operation, the input pump which is active at the respective input, regulates the pressure to the currently flow-determining balancing pump (1 or 2) of this channel to a constant value **in that**, in each case, a pressure sensor which is arranged between the two pumps controls the flow rate of the currently active input pump in order to keep the input pressure conditions of the flow-determining balancing pump (1, 2) in the channel equal and constant in both cycles.

7. Device according to claim 4 and 6, **characterised in that**, during operation, the output-side pumps (3) which are currently active in the cycle are also controlled by pressure sensors connected upstream at their inputs, so that the output pressure conditions of the flow-determining balancing pumps (1, 2) are also kept equal and constant in both cycles.

8. Device according to claim 4, **characterised in that**, during operation, cross-over pumping is achieved by specific control of the input pump of one channel and of the output pump of the other channel through a balancing pump, said cross-over pumping corresponding to a direct bypass function in all substance-exchange applications, since said cross-over pumping returns to the process exactly the liquid that was removed, without exchanging said liquid.

## Revendications

1. Procédé pour l'acheminement équilibré de deux quantités de liquide à l'aide d'un dispositif commun avec deux entrées et deux sorties et deux conduits de liquide (A, B) qui relient chacun une entrée à la sortie correspondante, étant précisé qu'une pompe d'équilibrage définissant le flux (1, 2) est disposée dans chacun des deux conduits de liquide (A, B), **caractérisé en ce qu'**il est prévu à chaque entrée et à chaque sortie de ces deux pompes d'équilibrage (1, 2) deux pompes à régulation de pression (3) qui sont reliées entre elles et commandées mutuellement de telle sorte que les pompes d'équilibrage (1, 2) définissent le flux dans l'un ou l'autre conduit et l'acheminent cycliquement, sélectivement, de l'une ou l'autre entrée du dispositif commun vers l'une ou l'autre sortie du dispositif commun, les pompes à régulation de pression (3) maintenant constantes les pressions d'entrée et de sortie des pompes d'équilibrage (1, 2).

**2.** Procédé selon la revendication 1, **caractérisé par** la commutation cyclique schématique des dispositifs à conduits dans une grille d'intervalles de même durée ou de même volume.

**3.** Procédé selon la revendication 1, **caractérisé par** une inversion symétrique de toutes les erreurs d'équilibrage totalisées précédemment et, de ce fait, par leur compensation dans le temps, grâce au fait que chaque commutation individuelle de conduit est réalisée dans une grille fixe de telle sorte que les affectations de conduits de toutes les commutations précédentes dans le temps soient effectuées encore une fois dans le même ordre, avec des conduits permutés.

**4.** Dispositif pour la mise en oeuvre du procédé selon la revendication 1, comprenant un dispositif commun avec deux entrées et deux sorties et deux conduits de liquide (A, B) qui relient chacun une entrée à la sortie correspondante, étant précisé qu'une pompe d'équilibrage définissant le flux (1, 2) est disposée dans chacun des deux conduits de liquide (A, B), **caractérisé en ce qu'**il est prévu à chaque entrée et à chaque sortie de ces pompes d'équilibrage (1, 2) deux pompes à régulation de pression (3) qui sont reliées entre elles et commandées mutuellement de telle sorte que les pompes d'équilibrage définissant le flux (1, 2) définissent de manière commutable, en fonctionnement, le flux dans l'un ou l'autre conduit et l'acheminent cycliquement, sélectivement, de l'une ou l'autre entrée du dispositif commun vers l'une ou l'autre sortie du dispositif commun, les pompes à régulation de pression (3) maintenant constantes les pressions d'entrée et de sortie des pompes d'équilibrage (1, 2).

**5.** Dispositif pour la mise en oeuvre du procédé selon la revendication 1, **caractérisé en ce que** les conduits de pompe, de liaison et de commutation des conduits de liquide (A, B) sont conçus comme un système de tuyaux flexibles et il est prévu comme pompes d'équilibrage (1, 2) des pompes à rouleaux.

**6.** Dispositif selon la revendication 4, **caractérisé en ce qu'**en fonctionnement, la pompe d'entrée qui est active au niveau de l'entrée correspondante règle à une valeur constante la pression vers la pompe d'équilibrage (1 ou 2) de ce conduit qui définit justement le flux, grâce au fait qu'un capteur de pression qui est disposé entre les deux pompes commande le débit de ladite pompe d'entrée justement active, afin de maintenir égales et constantes dans les deux cycles les conditions de pression d'entrée de la pompe d'équilibrage (1, 2) qui définit le flux dans le conduit.

**7.** Dispositif selon les revendications 4 et 6, **caractérisé en ce qu'**en fonctionnement, les pompes (3) prévues côté sortie qui sont justement actives dans le cycle sont commandées par des capteurs de pression montés en amont, au niveau de leurs entrées, de sorte que les conditions de pression de sortie des pompes d'équilibrage définissant le flux (1, 2) sont elles aussi maintenues égales et constantes dans les deux cycles.

**8.** Dispositif selon la revendication 4, **caractérisé en ce qu'**en fonctionnement, un acheminement croisé est obtenu grâce à une commande appropriée de la pompe d'entrée d'un conduit et de la pompe de sortie de l'autre conduit, à travers une pompe d'équilibrage, lequel acheminement croisé correspond dans toutes les applications de transfert de matière à une fonction de dérivation directe, étant donné qu'elle ramène exactement dans le processus le liquide qui a été prélevé, sans l'échanger.

1. Phase

2. Phase

Vom Pat.  Substituat

Vom Pat.  Substituat

A    B

A    B

3

3

1    2

1    2

3

3

B    A

B    A

Zum Pat.  Waste

Zum Pat.  Waste

Fig. 1

Fig. 2

Fig.3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 04267040 A
- EP 00867195 A1
- US 3620215 A
- US 5836908 A
- FR 2368963
- DE 3439661
- EP 0834329 A1
- DE 19856744